# EUROPEAN PATENT SPECIFICATION

(11) **EP 1 436 374 B1**
(45) Date of publication and mention of the grant of the patent: **20.08.2008**
(21) Application number: 02776238.4
(22) Date of filing: 18.10.2002
(51) Int. Cl.: C11D 3/50, C11D 3/37, C11D 3/30, A61Q 13/00

(54) **BENEFIT AGENT DELIVERY SYSTEMS**
WIRKSTOFFABGABESYSTEME
SYSTEMES D'ADMINISTRATION D'AGENT BENEFIQUE

(30) Priority: 19.10.2001 US 359644 P
(43) Date of publication of application: 14.07.2004
(73) Proprietor: THE PROCTER & GAMBLE COMPANY, Cincinnati, Ohio 45202 (US)
(72) Inventor: SMETS, Johan, B-3210 Lubbeek (BE); DYKSTRA, Robert, Richard, Cleves, OH 45002 (US); GRAY, Lon, Montgomery, Florence, KY 41042 (US); GALLON, Lois, Sara, Finneytown, OH 45231 (US); FREDJ, Abdennaceur, Loveland, OH 45140 (US); WHITE, Daniel, Jerome, Jr., West Chester, OH 45069 (US)
(74) Representative: Mather, Peter Geoffrey
(86) International application number: PCT/US2002/033378
(87) International publication number: WO 2003/033636

(56) References cited:
- EP-A- 0 971 025
- WO-A-00/55288
- WO-A-01/93823
- US-A- 3 939 099
- US-A- 4 511 495
- US-B1- 6 184 188

## Description

### Technical Field

The present invention relates to benefit agent delivery systems which can be used to deposit benefit agents such as perfumes onto the surface of a substrate, e.g., fabrics being laundered, hard surfaces, hair, or skin.

### Background of the Invention

It is frequently desirable or advantageous to treat the surfaces of a variety of substrates, for example fabrics, skin, hair, etc., with benefit agents such as perfumes, flavors, pharmaceuticals and/or biocontrol agents including biocides, insecticides, mildewcides, and the like. The objective of such treatment is generally to leave deposited on the surfaces of the substrates enough benefit agent so that there is a residual benefit imparted to the substrate surface after treatment of the substrate is completed.

Products, systems and methods for depositing benefit agents onto the surfaces of substrates are well known in the art. For example, in the context of fabric treatment such as fabric laundering, a variety of laundry and other products exist which can be used to form aqueous washing liquors or rinse baths containing benefit agents which deposit onto the surfaces of fabrics which are contacted with such liquors or baths.

One type of laundry product which involves the improved deposition of perfume materials onto fabrics laundered with such products is described in U.S. Patent No. 6,103,678. This '678 patent discloses laundry detergent or other treatment compositions which utilize the combination of an amino-functional polymer and a selected type of hydrophilic perfume in order to obtain effective perfume substantivity on fabrics laundered using such compositions,

Other types of products which provide improved deposition onto substrate surfaces of benefit agents such as perfumes are described in PCT Patent Application Nos. WO 00/02991;, WO 00/02981; WO 00/02987 and WO 00/02982. These patent publications disclose compositions wherein benefit agent substantivity on treated substrates is realized by incorporating into substrate treatment products a reaction product formed from amine-based compounds and certain types of, benefit agents which are pre-reacted with such amine-based compounds. US 3,939,099 discloses a fragrance composition comprising the combination of a fragrance oil and a film-former which are mutually soluble in a water-ethanol solvent.

However, notwithstanding the advances in the art as represented by the foregoing patent and patent publications, there remains a continuing need to identify benefit agent delivery systems which are especially effective for delivering residual and long-lasting benefit agents to substrates treated using such delivery systems.

### Summary of the Invention

The present invention provides a perfume delivery system suitable for delivering a perfume to the surface of a substrate according to Claim 1. Such a delivery system comprises a liquid or granular matrix to which is separately added both an amine-based compound and a perfume. The amine-based compound is a polyamine and will have a molecular weight greater than 100 Daltons. At least 10% of the amino groups of the amine-based compound must be primary amino groups.

The perfume and the separately added amine-based compound are selected and formulated together so that they will be exposed to and preferably deposit onto the surface of the substrate by means of contact of the substrate surface with a diluted, and preferably an aqueous, solution or dispersion of the delivery system. When this happens, the perfume will provide its benefit on and to the surface of the substrate for a longer period of time than when the amine-based compound is not present.

Most preferably the delivery system will comprise a liquid or granular laundry detergent or fabric-treating composition. The benefit agent which is delivered will be a perfume material. Compositions in the form of body wash products and shampoos are also contemplated.

### Detailed Description of the Invention

The essential components of the perfume delivery systems of the present invention include a liquid or granular matrix to which is separately added an amine compound and a perfume in certain chemical form. Each of these components is discussed in detail as follows along with other elements of the delivery systems herein as well as methods for their preparation and use.

### Liquid or Granular Matrix

The perfume delivery systems herein are based on the formation of a liquid or granular matrix. "Liquids" include fluids of density and viscosity which are conventional for liquids as well as gels and foams. Useful liquids may be aqueous or non-aqueous. Water is typically the major component of the delivery systems which are in aqueous liquid form. Conventional non-aqueous solvents may be used to form the matrix for liquid delivery systems in non-aqueous form. Liquid products, i.e., those containing 10% or greater of water or other solvents, are highly preferred.

Delivery systems in granular form can be fashioned from any type of solid-state material which comprises particles or granules ranging in size from 1 µm to 100 mm. Thus the granular matrix can include particles ranging from very fine powder to agglomerates or tablets. The granular matrix furthermore can comprise either inert or active ingredients, or both, with respect to the function of the product into which the delivery system is to be incorporated:

Most typically, the liquid or granular matrix used to form the delivery systems herein will comprise the matrix for the liquid or granular end product into which the benefit agent delivery systems will be incorporated and made a part of. Thus, for example, liquid or granular detergent compositions for laundry or hard surface cleaning will frequently comprise the liquid or granular matrix into which the amine-based compounds and benefit agents described herein will be separately added to form the delivery systems of this invention.

### Separate Addition of Amine Compound and perfume

It is an essential feature of the present invention that the amine compound and the perfume be added separately to the liquid or granular matrix. For purposes of this invention, the amine-based compound and perfume are separately added to the system-forming matrix if the entire amounts of these components are combined with the matrix as discrete components. In particular, there must be essentially no chemical reaction between these two materials before they are combined with the matrix. Thus the amine compound and the perfume may be added to the matrix at separate times and/or from separate containers or from separate holding or delivery means. The amine compound and the perfume materials may even be mixed together prior to combination with the system-forming matrix so long as substantially no chemical reaction occurs between these materials prior to their contact with the system-forming matrix.

### Amine-Based Compound

The amine-based compound which is added to the liquid or granular matrix as part of delivery system preparation will be a polyamine, the molecular weight of the compound will be at least 150 Daltons, and from 15% to 80% of its amino groups will be primary amino groups.

The amine-based compounds used in this invention are also preferably ones characterized by having an Odor Intensity Index of less than that of a 1% solution of methylanthranilate in dipropylene glycol.

### Odor Intensity Index Method

Odor Intensity Index is a value determined by expert graders who evaluate test chemicals for odor when such the pure chemicals are diluted at 1% in dipropylene glycol (DPG), odor-free solvent used in perfumery. This concentration percentage is representative of typical usage levels. Smelling strips, or so called "blotters", were are dipped in test solutions and presented to the expert panelist for evaluation. Expert panelists are assessors trained for at least six months in odor grading and whose grading are checked for accuracy and reproducibility versus a reference on an on-going basis. For each amine compound, a panelist is presented two blotters: one reference (Me Anthranilate, unknown from the panelist) and the test sample. The panelist is asked to rank both smelling strips on the 0-5 odor intensity scale, 0 being no odor detected, 5 being very strong odor present.

### Results:

The following represents Odor Intensity Index of some amine compounds suitable for use in the present invention and according to the above procedure. In each case, numbers are arithmetic averages among 5 expert panelists and the results are statistically significantly different at 95% confidence level:

| | |
|---|---|
| Methylanthranilate 1% (reference) | 3.4 |
| Ethyl-4-aminobenzoate (EAB) 1% | 0.9 |
| 1,4-bis-(3-aminopropyl)-piperazine (BNPP) 1% | 1.0 |

Amine compound suitable for use in the present invention are polyethyleneimine polymers, partially alkylated polyethylene polymers, polyethyleneimine polymers with hydroxyl groups, 1,5-pentanediamine, 1,6-hexanediamine, 1,3 pentanediamine, 3-dimethylpropanediamine, 1,2-cyclohexanediamine, 1,3-bis(aminomethyl)cyclohexane, tripropylenetetraamine, bis (3-aminopropyl)piperazine, dipropylenetriamine; tris(2-aminoethylamine), tetraethylenepentamine, bishexamethylenetriamine, bis(3-aminopropyl) 1,6 - hexamethylenediamine, 3,3'-diamino-N-methyldipropylamine, 2-methyl-1,5-pentanediamine, N,N,N',N'-tetra(2-aminoethyl)ethylenediamine, N,N,N',N'-tetra(3-aminopropyl)-1,4-butanediamine, pentaethylhexamine, 1,3-diamino-2-propyl-tert-butylether; isophorondiamine, 4,4',-diaminodicydohylmethane; N-methyl-N-(3-aminopropyl)ethanolaimine,spermine, spermidine, 1-piperazineethaneamine; 2-(bis(2-aminoethyl)amino)ethanol, ethoxylated N-(tallowalkyl)trimethylene diamines,poly[oxy(methyl-1,2-ethanediyl)], α-(2-aminomethyl-ethoxy)- (= C.A.S No. 9046-10-0); poly[oxy(methyl-1,2-ethanediyl)] α-hydro-)-ω-(2-aminomethylethoxy)-, ether with 2-ethyl-2-(hydroxymethyl)-1,3-propanediol (= C.A.S. No. 39423-51-3); commercially available under the tradename JeffaminesT-403, D-230, D-400, D-2000; 2,2',2"-triaminotriethylamine; 2,2'-diamino-diethylamine; 3,3'-diamino-dipropylamine, 1,3 bis aminoethyl-cyclohexane commercially available from Mitsubishi and the C12 Sternamines commercially available from Clariant like the C12 Sternamin(propylenamine)ₙ with n=3/4, and mixtures thereof. Preferred polyamines are polyethyleneimines commercially available under the tradename Lupasol like Lupasol FG (MW 800), G20wfv (MW 1300), PR8515 (MW 2000), WF (MW 25000), FC (MW 800), G20 (MW 1300), G35 (MW 1200), G100 (MW 2000), HF (MW 25000), P (MW 750000), PS (MW 750000), SK (MW 2000000), SNA (MW 1000000). Of these, the most preferred include Lupasol HF or WF (MW 25000), P (MW 750000), PS (MW 750000), SK (MW 2000000), 620wfv (MW 1300) and PR 1815 (MW 2000), Epomin SP-103, Epomin SP-110, Epomin SP-003, Epomin SP-006, Epomin SP-012, Epomin SP-018, Epomin SP-200, and partially alkoxylated polyethyleneimine, like polyethyleneimine 80% ethoxylated from Aldrich.

Also preferred compounds are the polyethylenimine dendrimers and the commercially available Starburst^{®} polyamidoamines (PAMAM) dendrimers, generation G0-G10 from Dendritech and the dendrimers Astromols^{®}, generation 1-5 from DSM being DiAminoButane PolyAmine DAB (PA)x dendrimers with x = 2ⁿ×4 and n being generally comprised between 0 and 4.

These most preferred amine compounds are selected from polyethyleneimine polymers commercially available under the tradename Lupasol like Lupasol HF, P, PS, SK, SNA, WF, G20wfv and PR8515; Even more preferred compounds are those selected from polyethyleneimine polymers having a molecular weight grater than 200 daltons including those commercially available under the tradename Lupasol like Lupasol HF, P, PS, SK, SNA, WF, G20wfv and PR8515;

### Benefit Agent

Another essential component of the perfume delivery systems herein is a perfume itself. The perfumes essentially used to form the delivery systems of this invention must be in the form of an aldehyde or ketone.

The perfume can, for example, be selected from a perfume ketone or aldehyde and mixtures thereof.

A typical disclosure of suitable ketone and/or aldehydes, traditionally used in perfumery, can be found in "Perfume and Flavor Chemicals", Vol. I and II, S. Arctander, Allured Publishing, 1994, ISBN 0-931710-35-5.

Perfume ketones are selected from Alpha Damascone, Delta Damascone, Iso Damascone, Beta-Ionone, Beta Damascone, and mixtures thereof.

Perfume aldehydes are selected from alpha-n-amyl cinnamic aldehyde, alpha-n-hexyl cinnamic aldehyde, P.T. Bucinal, lyral, cymal, lilial, Datilat, helional, triplal, melonal, and mixtures thereof.

### Indirect Application of Delivery Systems to Substrate Surfaces

The components of the benefit agent delivery systems herein are selected and processed such that the resulting delivery systems provide their benefit in a certain manner to substrate surfaces which have been indirectly contacted with such delivery systems. For purposes of this invention, indirect application of the delivery system occurs when a substrate surface is contacted with a dilute solution of the delivery system, such as in aqueous solution or dispersion of such a delivery system. For purposes of this invention, a "dilute" solution of the delivery system is a solution that contains a lower, i.e., less than 50%, concentration of the perfume when exposed to the substrate than was the concentration of the perfume in the delivery system prior to such exposure. For example, the perfume may be at one-half of the concentration it was in the delivery system in the aqueous solution or dispersion which is exposed to the substrate. Such aqueous solutions or dispersions can, of course, be formed by diluting the delivery system, or end product containing it, with water. This typically occurs when a product containing the delivery system is used for its intended purpose such as, for example, when a laundry detergent containing a perfume delivery system is used to launder fabrics. For purposes of this invention, an aqueous solution or dispersion of a delivery system is one which contains no more than 5000 ppm, preferably no more than 500 ppm, even more preferably no more than 50 ppm, and most preferably no more than 10 ppm and even sometimes no more than 1 ppm, of the benefit agent.

Indirect application of the delivery system includes any situation wherein the ultimate treatment of the substrate involved occurs with an aqueous solution or dispersion of the delivery system-containing product. This is true even if a substrate may initially be contacted with the concentrated delivery system-containing product. Thus, for example, even though a shampoo or body wash product may initially be contacted with and applied directly to hair or skin, such products are quickly diluted by the addition of water and used thereafter for indirct application of the benefit agent delivery system.

The materials used in the delivery systems are such that the system is especially effective for delivering the perfume to the surface of a substrate which has been indirectly contacted, i.e., via an aqueous solution or dispersion, with the product containing the delivery system. Under such conditions, the perfume delivered to the substrate surface will provide its benefit thereto for a longer period of time than if no amine-based compound were present in the delivery system. Of course, in determining such comparative delivery of perfume to a substrate surface, there must be sufficient contact of substrate with treating solution or dispersion in order to deposit at least some measurable amount of benefit agent on the surface.

### Delivery System Preparation

The benefit agent delivery system suitable for use in granular forms/matrices can be prepared by simply admixing the amine-based compound and the perfume ketone and/or aldehyde with the matrix under conditions which are sufficient to bring about combination, e.g., thorough admixture, of these components with the liquid or granular matrix. Frequently this admixing is carried out using high shear agitation. Temperatures of from 40°C to 65°C may be utilized. Additional materials may also be added to the matrix in order to form the complete end product into which the delivery system is to be incorporated.

In liquid matrices, especially, on a weight basis, the ratio of amine to perfume can vary widely, and will range 1000:1 to 1:50. In one embodiment, the ratio of amine to perfume is from 1000:1 to 1:5, more typically from 100:1 to 1:2, even more typically from 50:1 to 1:1, for the two essential components. (amine compound and ketone/aldehyde perfume. As noted, these two components do need to be added separately, i.e., in a form such that they are unreacted with each other. Thus these two components do not have to be added to the matrix simultaneously. They are, in fact, preferably added to the matrix sequentially.

### Cleaning and Fabric Treatment Products

The perfume delivery systems of the present invention are preferably incorporated into a wide variety of cleaning products and fabric treatment products. Such products include both laundry and cleaning compositions which are typically used for laundering fabrics and cleaning hard surfaces such as dishware, floors, bathrooms, toilet, kitchen and other surfaces in need of a prolonged or delayed release of the perfume. Accordingly, by laundry and cleaning compositions, these are to be understood to include not only detergent compositions which provide fabric cleaning benefits, but also compositions such as hard surface cleaning which provide hard surface cleaning benefit.

Products in which the delivery systems herein can be incorporated also include fabric treatment products such as fabric softeners or conditioners. Such products do not necessarily impart a cleaning benefit to fabrics treated therewith.

Preferred as products in which the delivery systems herein can be incorporated are those laundry and fabric treatment, e.g., softener, compositions which provide deposition of the perfume onto fabrics via contact of fabrics with aqueous solutions or dispersions of the products.

The effectiveness of the delivery to treated surfaces of the perfumes, can be quantified by means of a parameter called the Dry Surface Odor Index. Such a parameter is fully described in PCT Application No. WO 00/02982. Preferably, the perfume delivery systems herein which are incorporated into cleaning and fabric treatment products will provide a Dry Surface Odor Index of more than 5 and preferably at least 10.

Cleaning products incorporating the perfume delivery systems of the present invention may also take the form of shampoos or body wash compositions. With such products, the substrate being cleaned is, of course, hair or skin.

In general, the perfume delivery systems herein can be incorporated into cleaning or fabric treatment products herein such that levels of amine plus perfume range from 0.005% to 10% by weight, more preferably fom 0.005% to 5%, even more preferably from 0.02% to 0.5% by weight. For cleaning products, the amine plus perfume combination will generally be incorporated at concentrations of from 0.005% to 10% by weight, along with from 1% to 50% by weight of a surfactant. For fabric treatment products, the amine perfume combination will generally be incorporated at concentrations of from 0.005% to 5% by weight, along with from 1% to 50% by weight of a fabric softening or treating agent. The cleaning and fabric treatment products containing the delivery systems herein can comprise a wide variety of additional adjuvants which are conventional for use in products of these types. Extensive disclosure of such conventional adjuvants can be found in PCT Patent Application Nos. WO 00/02982 and WO 00/02987.

The cleaning and treatment products which contain the perfume delivery systems herein may take a variety of physical forms including liquids, gels or foams in aqueous or nonaquous form, granular form or tablet form. An especially preferred form for products of this type is a liquid detergent composition, e.g., a heavy duty liquid (HDL) detergent for fabric laundering.

Preparation of the perfume delivery systems herein and their incorporation into certain types of cleaning products can be illustrated by the following examples:

### EXAMPLE I

### Preparation of Liquid Detergent Composition

A heavy-duty liquid detergent composition in accordance with the present invention can be made as follows:
Step 1 ― a conventional heavy-duty liquid detergent composition is made by any conventional method known in the art;
Step 2 ― 0.01 % by weight of an amine in accordance with the present invention is added to the composition from Step 1 and the composition is then mixed for about 1-3 minutes;
Step 3 ― 0.015% by weight of a perfume in accordance with the present invention is added to the amine-containing composition from Step 2 and the composition is then mixed for about 5 minutes.
* Note that Step 2 and Step 3 are separate discrete addition steps.

A variety of detergent compositions are prepared having the compositions shown in the following Examples II through VI. In these examples the abbreviated component identifications have the following meanings:
- LAS:: Sodium linear Z12 alkyl benzene sulphonate
- CFAA:: C₁₂ - C₁₄ alkyl N-methyl glucamide
- HEDP :: Hydroxyethane dimethylene phosphonic acid
- DETPMP:: Diethylene triamine penta (methylene phosphonic acid), marketed by Monsanto under the Tradename Dequest 2060
- TEPAE: Tetreaethylenepentaamine ethoxylate
- PVP: Polyvinylpyrrolidone polymer
- PVNO: Polyvinylpyridine-N-Oxide, with an average molecular weight of 50,000.
- Brightener: Disodium 4,4'-bis(2-sulphostyryl)biphenyl and/or Disodium 4,4'-bis(4-anilino-6-morpholino-1.3.5-triazin-2-yl) stilbene-2:2'-disulfonate.

- Suds Suppressor-: 25% paraffin wax Mpt 50°C, 17% hydrophobic silica, 58% paraffin oil Granular suds suppressors12% Silicone/silica, 18% stearyl alcohol, 70% starch in granular form
- PEI: Polyethyleneimine
- Enzymes :: Protease, amylase, cellulase and/or lipase
- SRP: Anionically end capped poly esters.

- MEA: Monoethanolamine
- SCS: Sodium Cumene Sulfonate

Amine No. 1 - Lupasol WF (PEI of MW 25,000)
Amine No. 2 - Lupasol G35 (PEI of MW 1200)
Amine No. 3 - N,N'-bis-(3-aminopropyl)-1,3-propanediamine
Amine No. 4 - N,N'-bis-(3-aminopropyl)-ethylenediamine
Perfume No.1 - Delta-damascone
Perfume No.2 - melonal
Perfume No.3 - triplal
Perfume No.4 - helional

### EXAMPLE II

A heavy duty liquid (HDL) detergent composition is prepared containing a perfume delivery system prepared as in Example I. Such a liquid detergent composition has the following formula:

| **Ingredient** | **% by wt.** |
|---|---|
| Trisodium Citrate | 3.480 |
| C12-18 Real Soap | 3.241 |
| Ethanol | 2.500 |
| MEA | 1.500 |
| Ca Formate | 0.050 |
| Propylene Glycol | 4.440 |
| Na Formate | 0.103 |
| Borax Premix (38%) | 1.500 |
| Glycerin | 2.700 |
| NaOH | 0.837 |
| Hydrophilic Dispersant | |
| (PEI 189 E15-E18) | 0.650 |
| Protease | 0.032 |
| Cellulase | 0.001 |
| Mannanase | 0.004 |
| Amylase | 0.003 |
| Suds Suppressor | 0.010 |
| DTPA | 0.150 |
| Hydrophobic Dispersant (PEI 600 | |
| E20) | 1.290 |
| Perfume No.1 according to present invention | 0.020 |
| Amine No. 1 according to present invention | 0.0150 |
| Brightener | 0.125 |
| C12-14 Alkyl Dimethyl Amine Oxide (Amine Oxide) | 0.740 |
| C12-13 AE9 | 2.220 |
| C25AE1.1S Na Paste | 15.372 |
| NaLAS | 4.743 |
| Red HP Liquitint Dye | 0.002 |
| Additional Perfume | 0.280 |
| Water | 54.300 |

### EXAMPLE III

A heavy duty liquid (HDL) detergent composition is prepared containing a perfume delivery system prepared as in Example I. Such a liquid detergent composition has the following formula:

| **Ingredient** | **% by wt.** |
|---|---|
| Trisodium Citrate | 2.082 |
| C12-18 Real Soap | 0.548 |
| Ethanol | 1.340 |
| MEA | 1.150 |
| Ca Formate | 0.050 |
| Propylene Glycol | 2.500 |
| Na Formate | 1.000 |
| Glycerin | 0.350 |
| NaOH | 0.597 |
| Hydrophilic Dispersant (PEI 189 E15-E18) | 0.210 |
| Protease | 0.011 |
| Mannanase | 0.001 |
| Amylase | 0.002 |
| Suds Suppressor | 0.010 |
| Kathon | 0.001 |
| Hydrophobic Dispersant (PEI 600 E20) | 0.420 |
| Perfume No.1 according to present invention | 0.013 |
| Amine No. 2 according to present invention | 0.010 |
| Brightener | 0.040 |
| C12-13 AE9 | 1.450 |
| C25AE1.1S Na Paste | 10.173 |
| NaLAS | 3.098 |
| Liquitint Blue 65 | 0.016 |
| Additional Perfume | 0.260 |
| Water | 74.867 |

### EXAMPLE IV

### Liquid Detergent Composition

A heavy duty liquid (HDL) detergent composition is prepared containing a perfume delivery system prepared as in Example I. Such a liquid detergent composition has the following formula:

| Component | Wt. % |
|---|---|
| C₁₂₋₁₅ alkyl ether (2.5) sulfate | 19.0 |
| C₁₂₋₁₃ alkyl ethoxylate (9.0) | 2.00 |
| C₁₂-₁₄ glucose amide | 3.50 |
| Citric Acid | 3.00 |
| C₁₂₋₁₄ Fatty Acid | 2.00 |
| MEA | to pH 8 |
| Ethanol | 3.41 |
| Propanediol | 6.51 |
| Borax | 2.5 |
| PEI - Lupasol G (MW-100) | 0.00075 |
| Damascone | 0.01 |
| Dispersant | 1.18 |
| Na Toluene Sulfonate | 2.50 |
| Dye, Brighteners, Enzymes, Preservatives, Suds Suppressor, Other Minors, Water | Balance |
| 100% | |

### EXAMPLE V

### Liquid Detergent Composition

The following liquid detergent formulations are prepared according to the present invention :

| | **A** | **B** | **C** | **D** | **E** |
|---|---|---|---|---|---|
| LAS | 11.5 | 9.0 | - | 4.0 | - |
| C25E2.5S | - | 3.0 | 18.0 | - | 16.0 |
| C45E2.25S | 11.5 | 3.0 | - | 16.0 | - |
| C23E9 | - | 3.0 | 2.0 | 2.0 | 1.0 |
| C23E7 | 3.2 | - | - | - | - |
| CFAA | - | - | 5.0 | - | 3.0 |
| TopPalmKernel Fatty Acid | 2.0 | - | 2.0 | 0.5 | 2.0 |
| Citric (50%) | 6.5 | 1.0 | 2.5 | 4.0 | 2.5 |
| Ca and/or Ca formate | 0.6 | 0.7 | 0.2 | 0.05 | 0.05 |
| SCS | 4.0 | 1.0 | 3.0 | 1.2 | - |
| Borate | 0.6 | - | 3.0 | 2.0 | 3.0 |
| Na hydroxide | 6.0 | 2.0 | 3.5 | 4.0 | 3.0 |
| Ethanol | 2.0 | 1.0 | 4.0 | 4.0 | 3.0 |
| 1,2 Propanediol | 3.0 | 2.0 | 8.0 | 8.0 | 5.0 |
| Monoethanolamine | 3.0 | 1.5 | 1.0 | 2.5 | 1.0 |
| TEPAE | 2.0 | - | 1.0 | 1.0 | 1.0 |
| Enzymes | 0.03 | 0.01 | 0.03 | 0.02 | 0.02 |
| Amine No. 3 according to present invention | 0.015 | 0.0075 | 0.00375 | 0.2 | 0.045 |
| Perfume No. 2 according to present invention | 0.02 | 0.01 | 0.005 | 0.015 | 0.0075 |
| SRP | 0.2 | - | 0.1 | - | - |
| DTPA | - | - | 0.3 | - | - |
| PVNO | - | - | 0.3 | - | 0.2 |
| Brightener | 0.2 | 0.07 | 0.1 | - | - |
| Suds suppressor | 0.04 | 0.02 | 0.1 | 0.1 | 0.1 |
| Miscellaneous and water | Balance to 100% | | | | |

### EXAMPLE VI

### Liquid Detergent Composition

Heavy duty liquid fabric cleaning compositions in accordance with the invention are prepared as follows:

| | A | B |
|---|---|---|
| LAS acid form | - | 25.0 |
| Citric acid | 5.0 | 2.0 |
| 25AS acid form | 8.0 | - |
| 25AE2S acid form | 3.0 | - |
| 25AE7 | 8.0 | - |
| CFAA | 5 | - |
| DETPMP | 1.0 | 1.0 |
| PEI - Lupasol PR8515 (MW-2000) 0.1 | | 0.06 |
| Damascone | 0.02 | 0.015 |
| Lilial | 0.06 | 0.05 |
| Fatty acid | 8 | - |
| Oleic acid | - | 1.0 |
| Ethanol | 4.0 | 6.0 |
| Propanediol | 2.0 | 6.0 |
| Coco-alkyl dimethyl | - | 3.0 |
| hydroxy ethyl ammonium chloride | | |
| Smectite clay | - | 5.0 |
| PVP | 2.0 | - |
| Water / Minors | Up to 100% | |

### EXAMPLE VII

### Liquid Detergent Composition

Heavy-duty liquid fabric cleaning compositions in accordance with the invention are prepared as follows:

| | A | B | C |
|---|---|---|---|
| C25AES | 18.0 | 15.0 | 14.0 |
| LAS | 5.8 | 5.0 | 4.0 |
| C₈₋₁₀ Amine | 1.4 | 2.0 | - |
| Nonionic 24-7 | 2.8 | 2.0 | 3.0 |
| Citric acid | 2.5 | 3.0 | 3.0 |
| Fatty acid | 8.5 | 3.0 | 3.0 |
| Enzymes | 0.02 | 0.02 | 0.006 |
| Boric acid | 2.0 | 2.0 | 2.0 |
| Ethoxylate tetraethylene pentaimine | 0.9 | 1.0 | 1.0 |
| Polyethylene imine ethoxylated | 0.7 | - | 1.0 |
| DETPMP | 0.3 | - | - |
| PEI - Lupasol P (MW-750,000) | 0,04 | 0.1 | 0.044 |
| Damascone | 0.02 | 0.02 | - |
| Lilial | 0.02 | 0.02 | - |
| Hexyl Cinnamic Aldehyde | - | 0.01 | 0.02 |
| Florhydral | - | - | 0.05 |
| HEDP | 0.35 | - | - |
| Ethanol | 1.0 | 3.0 | 3.0 |
| 1,2,propanediol | 8.0 | 4.0 | 5.0 |
| MEA | 9.8 | 2.0 | 2.0 |
| Na Cumene Sulfonate | 2.0 | - | - |
| Suds suppressors | 0.25 | 0.01 | 0.01 |
| Minors (perfumes, brighteners) and water | Up to 100% | | |

### EXAMPLE VIII

### Granular Detergent Composition

A heavy duty granular detergent (HDG) composition is prepared containing the pro-perfume composition of Example I. Such a granular detergent composition has the following formula:

| Component | Wt. % |
|---|---|
| C₁₂ Linear alkyl benzene sulfonate | 9.31 |
| C₁₄₋₁₅ alkyl sulfonate | 12.74 |
| Zeolite Builder | 27.79 |
| Sodium Carbonate | 27.31 |
| PEG 4000 | 1.60 |
| Dispersant | 2.26 |
| C₁₂₋₁₃ alkyl ethoxylate (E9) | 1.5 |
| Sodium Perborate | 1.03 |
| Soil Release Polymer | 0.41 |
| PEI - Lupasol SK (MW-2,000,000) | 0.04 |
| Damascone | 0.02 |
| Lilial | 0.03 |
| Florhydral | 0.01 |
| Enzymes | 0.59 |
| Brightener, Suds Suppressor, Other Minors, Moisture | 0.3 |
| Sulfate | Balance up |
| | to 100% |

### EXAMPLE IX

| Body Wash | | | |
|---|---|---|---|
| Ingredient | A | A | A |
| Sodium Laureth Sulfate | 7.5 | 8.5 | 8.2 |
| Cocamidopropyl Betaine | 6.5 | 5.5 | 4.5 |
| Sodium Lauroyl Sarcosinate | 0.75 | 0.65 | 1.2 |
| Citric Acid | 0.26 | 0.33 | 0.38 |
| Guar Hydroxypropyltrimonium Chloride | 0.50 | 0.30 | 0.30 |
| Lauryl Alcohol | 0.65 | 0.80 | 0.77 |
| DMDM Hydantoin | 0.21 | 0.26 | 0.11 |
| Sodium Benzoate | 0.25 | 0.15 | 0.18 |
| Disodium EDTA | 0.10 | 0.05 | 0.20 |
| Amine No. 3 according to present invention | 1.8 | 0.8 | 0.35 |
| Amine No. 4 according to present invention | -- | -- | 0.15 |
| Perfume No. 3 according to present invention | 0.7 | 2.1 | 1.1 |
| Water/Carriers/aesthetics | balance | balance | balance |

### EXAMPLE X

| Shampoo | | | |
|---|---|---|---|
| Ammonium Laureth / Lauryl Sulfate | 16 | 14 | 20 |
| Glycol Distearate | 1.5 | 1.1 | 1.6 |
| Dimethicone | 1.4 | 1.1 | 1.8 |
| Cetyl Alcohol | 0.90 | 1.2 | 1.4 |
| Cocamide MEA | 0.75 | 0.95 | 0.55 |
| Sodium Chloride | 0.65 | 1.0 | 1.3 |
| Polyquaternium-10 (LR-400) | 0.50 | 0.30 | 0.20 |
| Sodium Citrate | 0.60 | 0.40 | 0.50 |
| Hydrogenated Polydecene | 0.30 | 0.20 | 0.70 |
| Sodium Benzoate | 0.20 | 0.35 | 0.40 |
| Disodium EDTA | 0.12 | 0.085 | 0.15 |
| Trimethylolpropane Tricaprylate/Tricaprate | 0.10 | 0.15 | 0.10 |
| Citric Acid | 0.040 | 0.050 | 0.040 |
| Pro vitamins | 0.060 | -- | 0.030 |
| Methylchloroisothiazolinone/ | 0.00038 | 0.0010 | 0.00031 |
| Methylisothiazolinone | 0.00012 | 0.00018 | 0.00028 |
| Amine according to present invention | 1.0 | 0.65 | 0.10 |
| perfume according to present invention | 0.50 | 0.75 | 1.2 |
| Water / Carriers / Aesthetics | Balance | balance | balance |

## Claims

1. A perfume delivery system suitable for delivering a perfume to the surface of a substrate, which perfume delivery system comprises a granular or liquid composition matrix for laundering or treating fabrics or cleaning hard surfaces, hair or skin, to which is separately added:
A) an amine compound selected from polyethyleneimines having a molecular weight greater than 150 Daltons, and having at least about 10% of its amino groups in the form of primary amino groups; and
B) a perfume selected from Damascone, alpha-Damascone, beta-Damascone, delta-Damascone, iso-Damascone, beta-Ionone, lilial, alpha-n-hexylcinnamic aldehyde, alpha-n-amylcinnamic aldehyde, cymal, lyral, butylcinnamic aldehyde, datilat, helional, triplal, melonal, and mixtures thereof;
in a weight ratio of amine compound to perfume ranging from 1000:1 to 1:50;
and wherein when said amine compound and said perfume are exposed to and preferably deposited onto a substrate surface via contact of said surface with an aqueous solution or dispersion of said delivery system, the perfume provides its benefit to said surface for a longer period of time than when said amine compound is not present.

2. A delivery system according to Claim 1 wherein the amine-based compound is a polyamine having a molecular weight of at least 150 Daltons and further having from 15% to 80% of its amino groups as primary amino groups.

3. A delivery system according to Claim 1 or Claim 2 wherein said amine-based compound has an Odor Intensity Index of less than that of a 1% solution of methylanthranilate in dipropylene glycol.

4. A cleaning or' fabric treatment product containing from 0.005% to 10%, preferably from 0.005% to 2%, by weight of a perfume delivery system according to any of Claims 1 to 3.

5. A cleaning composition comprising from 1 % to 50% by weight of a surfactant and from about 0.005% to 10% by weight of a perfume delivery system according to Claim 1.

6. A cleaning composition according to Claim 5 which is in the form of a liquid detergent composition.

7. A cleaning composition according to Claim 5 which is in the form of a shampoo or body wash.

8. A fabric treatment composition comprising from 1% to 50% by weight of a fabric softening or treatment agent and from 0.005% to 5% of a perfume delivery system according to any of Claims 1 to 3.

## Patentansprüche

1. Duftstoffabgabesystem, das zum Abgeben eines Duftstoffs an die Oberfläche eines Substrats geeignet ist, wobei das Duftstoffabgabesystem eine granuläre oder flüssige Zusammensetzungsmatrix zum Waschen oder Behandeln von Textilien oder zum Reinigen von harten Oberflächen, Haaren oder Haut umfasst, zu der
A) eine Aminverbindung, ausgewählt aus Polyethyleniminen mit einem Molekulargewicht von über 2,49E-22 g (150 Dalton), deren Aminogruppen zu mindestens etwa 10 % in Form von primären Aminogruppen vorliegen; und
B) ein Duftstoff, ausgewählt aus Damascon, alpha-Damascon, beta-Damascon, delta-Damascon, iso-Damascon, beta-Ionon, Lilial, alpha-n-Hexylzimtsäurealdehyd, alpha-n-Amylzimtsäurealdehyd, Cymal, Lyral, Butylzimtsäurealdehyd, Datilat, Helional, Tripial, Melonal und deren Mischungen separat zugegeben werden;
in einem Gewichtsanteil von Aminverbindung zu Duftstoff, das im Bereich von 1000:1 bis 1:50 liegt;
und wobei, wenn die Aminverbindung und der Duftstoff einer Substratoberfläche ausgesetzt und vorzugsweise auf diese aufgebracht werden, **dadurch**, dass eine Oberfläche mit einer wässrigen Lösung oder Dispersion des Abgabesystems in Kontakt gebracht wird, der Duftstoff der Oberfläche über einen längeren Zeitraum als wenn die Aminverbindung nicht vorhanden wäre einen Vorteil verleiht.

2. Abgabesystem nach Anspruch 1, wobei die Verbindung auf Aminbasis ein Polyamin mit einem Molekulargewicht von mindestens 2,49E-22 g (150 Dalton) ist und ihre Aminogruppen ferner zu 15 % bis 80 % primäre Aminogruppen sind.

3. Abgabesystem nach Anspruch 1 oder Anspruch 2, wobei die Verbindung auf Aminbasis einen Geruchsintensitätsindex von weniger als dem einer 1 %-igen Lösung von Methylanthranilat in Dipropylenglycol aufweist.

4. Reinigungs- oder Textilbehandlungsprodukt, das zu 0,005 Gew.-% bis 10 Gew.-%, vorzugsweise zu 0,005 Gew.-% bis 2 Gew.-% ein Duftstoffabgabesystem gemäß einem der Ansprüche 1 bis 3 enthält.

5. Reinigungszusammensetzung, die zu 1 Gew.-% bis 50 Gew.-% ein Tensid und zu etwa 0,005 Gew.-% bis 10 Gew.-% ein Duftstoffabgabesystem gemäß Anspruch 1 umfasst.

6. Reinigungszusammensetzung nach Anspruch 5, die in Form einer flüssigen Detergenszusammensetzung vorliegt.

7. Reinigungszusammensetzung nach Anspruch 5, die in Form eines Shampoos oder eines Körperreinigungsmittels vorliegt.

8. Textilbehandlungszusammensetzung, die zu 1 Gew.-% bis 50 Gew.-% einen Textilweichmacher oder ein Textilbehandlungsmittel und zu 0,005 Gew.-% bis 5 Gew.-% ein Duftstoffabgabesystem gemäß einem der Ansprüche 1 bis 3 umfasst.

## Revendications

1. Système de libération de parfum approprié pour libérer un parfum sur la surface d'un substrat, lequel système de libération de parfum comprend une matrice de composition granulaire ou liquide pour laver ou traiter des tissus ou nettoyer des surfaces dures, des cheveux ou la peau, auquel est ajouté séparément :
A) un composé aminé choisi parmi les polyéthylène-amines ayant une masse moléculaire supérieure à 2,49E-22 g (150 Daltons), et ayant au moins environ 10 % de ses groupes amino sous la forme de groupes amino primaires ; et
B) un parfum choisi parmi Damascone, alpha-Damascone, bêta-Damascone, delta-Damascone, iso-Damascone, bêta-Ionone, lilial, aldéhyde alpha-n-hexylcinnamique, aldéhyde alpha-n-amylcinnamique, cymal, lyral, aldéhyde butylcinnamique, datilat, hélional, tripial, melonal, et leurs mélanges ;
dans un rapport pondéral de composé aminé allant de 1000:1 à 1:50 ;
et dans lequel lorsque ledit composé aminé et ledit parfum sont exposés à et de préférence déposés sur une surface de substrat via un contact de ladite surface avec une solution ou dispersion aqueuse dudit système de libération, le parfum fournit son effet bénéfique sur ladite surface pendant une plus longue période de temps que lorsque ledit composé aminé n'est pas présent.

2. Système de libération selon la revendication 1, dans lequel le composé à base d'amine est une polyamine ayant une masse moléculaire d'au moins 2,49E-22 g (150 Daltons) et ayant, en outre, de 15 % à 80 % de ses groupes amino en tant que groupes amino primaires.

3. Système de libération selon la revendication 1 ou la revendication 2, dans lequel ledit composé à base d'amine a un indice d'intensité d'odeur de moins de celui d'une solution à 1 % de méthylanthranilate dans du dipropylène glycol.

4. Produit de nettoyage ou de traitement des tissus contenant de 0,005 % à 10 %, de préférence de 0,005 % à 2 % en poids d'un système de libération de parfum selon l'une quelconque des revendications 1 à 3.

5. Composition de nettoyage comprenant de 1 % à 50 % en poids d'un agent tensioactif et d'environ 0,005 % à 10 % en poids d'un système de libération de parfum selon la revendication 1.

6. Composition de nettoyage selon la revendication 5, qui est sous la forme d'une composition détergente liquide.

7. Composition de nettoyage selon la revendication 5, qui est sous la forme d'un shampooing ou d'un produit de lavage pour le corps.

8. Composition de traitement des tissus comprenant de 1 % à 50 % en poids d'un agent d'adoucissement ou de traitement des tissus et de 0,005 % à 5 % d'un système de libération de parfum selon l'une quelconque des revendications 1 à 3.
